# EUROPEAN PATENT APPLICATION

(11) **EP 2 837 346 A1**
(43) Date of publication of application: **18.02.2015**
(21) Application number: 13755839.1
(22) Date of filing: 25.02.2013
(51) Int. Cl.: A61B 17/64, A61B 17/88

(54) **EXTERNAL FIXATOR FOR PELVIC FRACTURES AND TURNBUCKLE FOR OPENING SAID FIXATOR**

(30) Priority: 01.03.2012 ES 201230223; 02.03.2012 ES 201230228
(71) Applicant: Universidad De Malaga, E-29071 Málaga (ES); Servicio Andaluz de Salud, 41071 Sevilla (ES)
(72) Inventor: QUEIPO DE LLANO TEMBOURY, Alfonso, 41071 - Sevilla (ES); ESQUERRO JUANCO, Francisco, 29071 Málaga (ES); PÉREZ DE LA BLANCA COBOS, Ana, 29071 Málaga (ES)
(74) Representative: Hoffmann Eitle
(86) International application number: PCT/ES2013/000051
(87) International publication number: WO 2013/128046

(57) **Abstract**

The invention describes an external fixator formed by a curved elastic bar with an essentially C shape which serves to immobilize pelvic fractures. It also describes a turnbuckle for opening said external fixator comprising two side support elements for the ends of the external fixator and a central support element for the central part of the external fixator, where the central support element is longitudinally movable along a line perpendicular to the line joining said side support elements.

## Description

### OBJECT OF THE INVENTION

The invention is comprised within the field of medicine, and more specifically in the field of pelvic fracture treatment.

An object of the invention is a novel external fixator formed essentially by a curved elastic bar which serves to immobilize pelvic fractures in a manner that is less cumbersome and more comfortable for patients compared with devices used today.

Another object of the invention is a novel turnbuckle particularly designed for opening the described external pelvic fixator in a controlled manner.

### PRIOR ART

The pelvis, formed by the two iliac bones joined at the front by the symphysis pubis and at the back to the sacrum has utmost functional importance. It transmits the load from the rachies to the lower limbs, from the sacroiliac joint, having a strong ligamentous complex. Strong muscles involved in posture and gait originate in the pelvis.

Due to the functional importance of this skeletal segment, the fracture thereof can cause significant functional disorders such as dysmetries, post-traumatic sacroiliac joint arthrosis, limp and disability, and impaired birth canal in women.

Most pelvic fractures are a clinical and therapeutically complex problem, particularly those involving pelvic ring rupture due to the hemorrhagic problem they entail. A rigid immobilizer or external fixator which is a device or structure located outside the skin stabilizing bone fragments connected by means of the so-called Schanz pins is commonly used to treat pelvic fractures. Given its simplicity and ease of placement, the external pelvic fixator is an instrument of utmost importance for emergency treatment of patients with such serious injury and is within all resuscitation protocols.

However, external fixators used today do not suitably stabilize bone fragments. Furthermore, they are very bulky apparatuses that are uncomfortable to use.

### DISCLOSURE OF THE INVENTION

A first aspect of the present invention solves the preceding problems as a result of a novel device based on an elastic bar with an essentially C shape at the ends of which Schanz pins are fixed, which pins are in turn fixed to the pelvic fragments to be stabilized. A significant advantage of this fixator consists of using its elastic properties to apply on the pelvic fragments constant tension which tends to keep the fragments in place when this fixator has been previously tensed. This elastic tension is a huge improvement in relation to earlier rigid devices, since the pelvic bone fragments are subjected to tension that tends to reposition them in the correct place, thus facilitating suitable bone fragment consolidation.

Therefore, in order to use the device of the invention it is only necessary to open the arms of the elastic bar with a C shape and fix Schanz pins to the bar and to the bone fragments of the patient in this situation. Once this has been performed, tension is no longer applied to the elastic bar which, due to its tendency to return to its original shape, creates a tension that is transmitted to the bone fragments through the Schanz pins, thus helping in stabilizing the pelvis of the patient.

However, the described elastic bar with a C shape is a device which is still somewhat cumbersome and uncomfortable for the patient who must remain still with said device installed on him/her for several days. For that reason, in order to reduce the size of the member which is fixed to the patient, in a preferred embodiment of the invention the device is formed by a bar with an essentially C shape having a curved elastic central portion and two end portions, where the end portions are connected to the central portion by means of a detachable joint allowing transmission of tensions between said end portions and the central portion. The ends can therefore be used for tensing the central portion more easily and, once the central portion has been tensed, the Schanz pins can be connected thereto. The end portions can then be detached to improve patient's comfort.

In principle, the end portions can have different shapes, sizes and materials provided that they allow gripping same and applying sufficient force thereon such as to cause tensing of the central portion. However, according to a preferred embodiment of the invention the end portions are curved, being a natural continuation of the central portion, for example, for forming a complete fixator with an essentially semicircular shape. Another possible option is that the end portions are straight.

According to another preferred embodiment, the elastic central portion is made of carbon fiber. As regards the end portions, they do not have to be of the same material as the central portion, being able to be made of carbon fiber, surgical steel or another material.

The joints between the central portion and the end portions can be configured in different ways provided that they are capable of transmitting tension and are easily and rapidly detachable. For example, the detachable joint can comprise first essentially planar prolongations projecting from the central portion which are complementary with second essentially planar complementary prolongations projecting from the end portions, where the essentially planar surface of the first prolongations and second prolongations are perpendicular to the curvature of the fixator. Since they are perpendicular and if they are long enough, suitable transmission of tension will be achieved with this joint. It must be noted that in this regard bending of the bar will occur within its own plane, and therefore always perpendicular to its curvature, i.e., perpendicular to the essentially planar surfaces. This will be seen more clearly in the detailed disclosure of the invention where references are made to the drawings.

Furthermore, the planar prolongations comprise radial holes which allow inserting fixing means, such as bolts, screws or the like.

Another example of a joint between the central portion and the end portions consists of a male-female joint where a longitudinal projection of the central portion or side portions fits into a corresponding longitudinal hole of the side portions or central portion, respectively. Said portions could also be fixed to one another by means of radial through bolts or screws, or the projection and the hole can be coupled to one another by means of threading. An outer sleeve can also be passed through the area to reinforce this coupling.

In summary, the installation of this novel fixator in its detachable configuration would be carried out in the following manner. Firstly, Schanz pins are inserted in the bone of the patient in the suitable positions according to the fracture. Next, after the fixator has been assembled with its central portion suitably fixed to the end portions, suitable force is applied to said end portions to bend the central portion, such that the curvature thereof increases. After the central portion has been tensioned, the Schanz pins are fixed to said central portion by means of suitable ball and socket joints. Next, the tension of the end portions is released such that the central portion, due to its tendency to return to its initial position, applies a force on the Schanz pins tending to move the bone fragments to which the pins are coupled closer together, said fragments being suitably located to achieve rapid consolidation. Finally, the end portions of the fixator are detached, only the central portion remains.

In order to use this novel fixator both in its configuration as a single elastic bar with a C shape and in its detachable configuration formed by a central portion and two end portions, it is necessary to open the arms of the elastic bar, overcoming the resistance applied by the latter. This step can be carried out manually in certain situations. However, there are cases in which it is essential to have greater control over the movement of the ends of the fixator, or in which the force to be applied is too much to manually apply the force with ease. Sometimes it would also be desirable to force the arms of the elastic bar in a controlled manner until exceeding the elastic limit of the material, thus being able to "open" or "close" them to adjust the distance between their ends according to each particular case.

A second aspect of the present invention describes a turnbuckle particularly designed for opening an elastic external fixator with an essentially C shape such as that described above. This turnbuckle essentially comprises two side support elements for the ends of the external fixator and a central support element for the central part of the external fixator, where the central support element is longitudinally movable along a line perpendicular to the line joining said side support elements. Therefore, by placing the external fixator with the inner part of its ends supported on the two side support elements, when the central support element moves longitudinally it pushes the outer part of the central area of the fixator, thus opening it.

This configuration can be implemented in different manners by using various mechanisms, although the opening turnbuckle described in the present document preferably comprises three members, a brace strut, two side members and a central member. Each of these members will be described in more detail below:
- Brace strut
   It is a strut to which the rest of the members forming the turnbuckle of the invention are coupled. Preferably, the brace strut is simply a straight bar. However, it is understood that other configurations are possible. For example, the brace strut could also be configured as a curved bar, for example, with a curvature similar to that of the external fixator such that, when the turnbuckle is in use, it would be located approximately parallel to said external fixator.
- Side arms
   The side arms can be slidably coupled to the brace strut and are provided with respective side support elements for the ends of the external fixator. Since the arms are sliding arms, the position of the side supports can be regulated for using the turnbuckle with external fixators of different sizes.
   More specifically, in a preferred embodiment of the invention the two side arms are two bars having a first end slidably coupled to said brace strut and a second end provided with the side support element. The side arms are preferably straight bars which are usually perpendicular to the brace strut, although other options where the side arms have another configuration are possible.
   In another preferred embodiment of the invention, the side arms further comprise means for regulating their position along the central strut. These means could be configured in different manners, such as a threading mechanism operated by means of a rotary control or key, for example. In any case, these means would allow the turnbuckle of the invention to not only "open" the external fixator at hand, but also to "close" it. This may be useful in cases in which it is necessary to move the ends of an external fixator closer, which could be achieve by "closing" it until exceeding the elastic limit.
   The side supports are preferably formed by pulleys allowing the external fixator supported thereon to slide smoothly without friction.
- Central member
   This member is coupled to the brace strut between the two side arms and has a central support element longitudinally movable along a line perpendicular to the line joining the side support elements.

The central member preferably comprises a straight hollow bar inside which a rod provided at the end thereof with the central support element can slide longitudinally operated by means of a rotary control. This central member is usually perpendicular to the brace strut.

Like the side supports, the central support is preferably provided with a pulley which minimizes friction during sliding of the external fixator.

The described opening turnbuckle is used in the manner described below. Firstly, the external fixator with a C shape is suitably placed, i.e., with the outer part of its central part supported on the central support element and the inner part of its ends supported on the side support elements. In this position, the central support element moves in a controlled manner perpendicular to the line joining the two side support elements. Since the external fixator having an essentially semicircular shape is supported at its ends on the side support elements, pushing its central part in causes the "opening" of said fixator.

By regulating the distance the central support element travels, the external fixator can be elastically "opened" for use in fixing a pelvic fracture (method that will be described in greater detail in reference to the drawings). Another option is by moving the central support element until exceeding the elastic limit of the fixator, thus achieving the practical effects of a more open external fixator, i.e., with more separated ends.

Similarly, if the arms are provided with means which allow regulating their position along the central strut in a controlled manner, for example, means similar to those used for moving the central support element, an equivalent method could be carried out to "close" the external fixator. In other words, the external fixator could be placed with the inner part of its central area supported on the central support element and the outer part of its ends supported on the side support elements. Therefore, by moving the arms towards one another, the side support elements "compress" the ends of the external fixator. Like the preceding case, the process can be stopped before reaching the elastic limit or exceeding the elastic limit to attain permanent deformation of the external fixator, for example, to achieve the practical effects of an external fixator that is "closed" to a greater extent that the original fixator.

### BRIEF DESCRIPTION OF THE DRAWINGS

Figure 1 shows a schematic view of an example of an external fixator formed by a single member according to the first aspect of the present invention.
Figure 2 shows a schematic view of an example of a detachable external fixator according to the first aspect of the present invention provided with curved end portions.
Figure 3 shows a schematic view of another example of a detachable external fixator according to the first aspect of the present invention provided with straight end portions.
Figure 4 shows a perspective view of an example of joint between a central portion and an end portion of a detachable fixator.
Figure 5 shows a perspective view of an opening turnbuckle according to the second aspect of the present invention.
Figures 6a-6c schematically show the steps necessary for installing the non-detachable fixator of Figure 1 using the opening turnbuckle of Figure 5.
Figures 7a-7d schematically show the steps necessary for installing the detachable fixator of Figure 4 using the opening turnbuckle of Figure 5.

### PREFERRED EMBODIMENT OF THE INVENTION

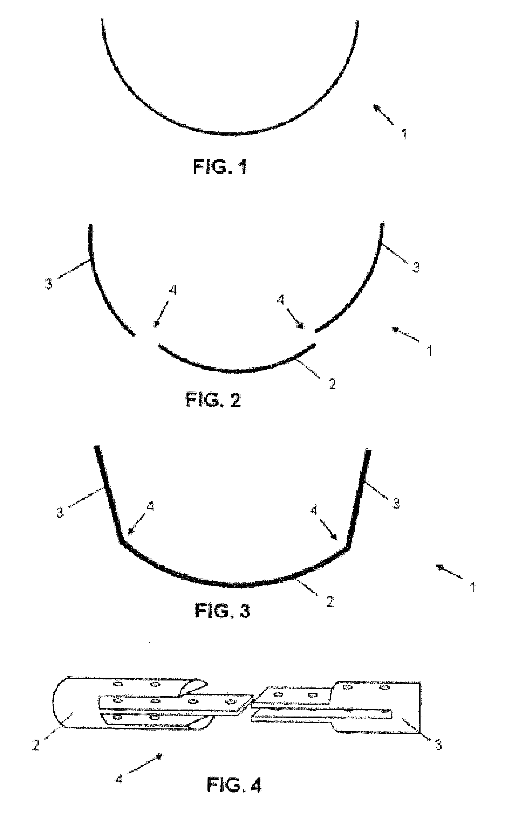

Figure 1 shows an example of a non-detachable external fixator (1) according to the first aspect of the present invention. This fixator (1) is formed by a single elastic bar the use of which will be described below in the present document.

Figure 2 shows an example of a detachable fixator (1) comprising a curved elastic central portion (2) and also curved end portions (3), such that the detachable joint (4) is closed resulting in a curved bar with a constant curvature forming a C or semicircle shape similar to that which is shown in Figure 1.

Figure 3 shows a second example of detachable fixator (1) where the end portions (3) are straight. Though not shown in the drawings, the detachable fixators (1) shown in the drawings have circular cross-section. However, it is understood that other shapes, such as for example square or the like, would be possible.

Figure 4 shows a detail of an example of a detachable joint (4) of a fixator (1) having a circular section and with curved end portions (3) showing how one of the ends of the central portion (2) is split longitudinally such that first planar prolongations (4a) are formed, whereas the end of an end portion (3) has complementary second planar prolongations (4b). The manner in which the prolongations fit into one another can be readily seen such that, when the joint (4) is fixed, for example by means of screws or the like through the holes (4c) arranged in the first prolongations (4a) and second prolongations (4b), the visual effects are similar to the fixator (1) of Figure 1 formed by a single bar without connections. Furthermore, these connections allow transmission of tension along the entire bar.

In this context, the term "essentially planar" does not refer to all the surfaces of the prolongations being planar. As seen in the drawing, the outer surface thereof is curved such that it continues with the outer profile of the circular cross-section of the fixator (1). Furthermore, though not seen in the drawings, the prolongations (4a, 4b) could include on their inner faces longitudinal flanges or grooves aiding suitable placement. Therefore, the term "essentially planar" covers all these possibilities.

In summary, this joint (4) is designed for suitably transmitting tension when the end portions (3) are "gripped" and tension tending to "open" the central portion (2) is applied. In fact, when placing the fixator (1), tension will be applied to the central portion (2) of the fixator tending to "open" it within its own plane, perpendicular to its curvature. Therefore, the essentially planar prolongations (4a, 4b) are applied by transverse bending perpendicular to said planar surfaces, thus achieving suitable transmission of tension.

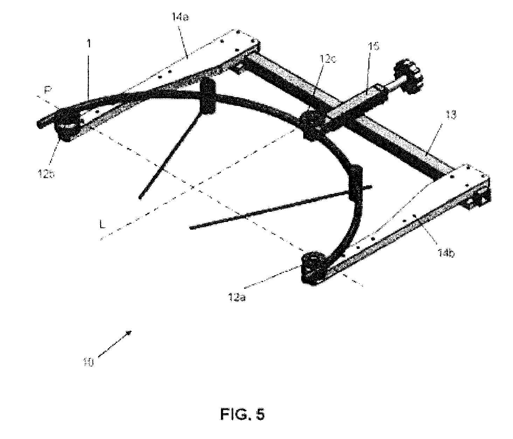

Figure 5 shows an example of a turnbuckle (10) according to the second aspect of the present invention. It shows how the essential elements of the turnbuckle (10) are the three support elements, the two side elements (12a, 12b) and the central element (12c); where the central support element (12c) can be moved longitudinally along a line (L) which is perpendicular to the line (P) joining the two side support elements (12a, 12b).

In this example, those movements are achieved by using a turnbuckle (10) having a central strut (13), consisting essentially of a straight bar having a square section, at the end of which the first ends of a pair of arms (14a, 14b) are slidably coupled. When the arms (14a, 14b) are said to be "slidably" coupled, it means that the arms can be moved longitudinally along said central strut (13) for being located in different positions along said strut (13). It is also understood that the first ends of the side arms (14a, 14b) have means for fixing their position, for example, either by using screws that are threaded directly to the central strut (13) or by means of a clamping mechanism.

The side support elements (12a, 12b) are coupled to the second ends of the side arms (14a, 14b). This example shows how the side support elements (12a, 12b) are formed by members in the form of a pulley that will allow sliding of the external fixator (1), which is of a single piece in this drawing.

A central member (15) is also coupled to the central strut (13), which member in this example is formed by a straight hollow bar coupled perpendicular to the central strut (13), said hollow bar having a rod which can be moved longitudinally through the inside thereof. A rotary control coupled to a simple threading mechanism (not shown in the drawings) is used to control movement. The central support element (12c) is arranged at the end of the rod, which element can therefore also move longitudinally perpendicular to the line joining the side support elements (12a, 12b).

This novel turnbuckle (10) works as follow. Firstly, the external fixator (1) is placed such that the outer part of its central part is supported on the central support element (12c), whereas the inner parts of its ends are supported on the side support elements (12a, 12b). Next, the rotary control of the central member (15) is simply operated to move the central support element (12c) forward in a controlled manner, thus causing the "opening" of the external fixator (1).

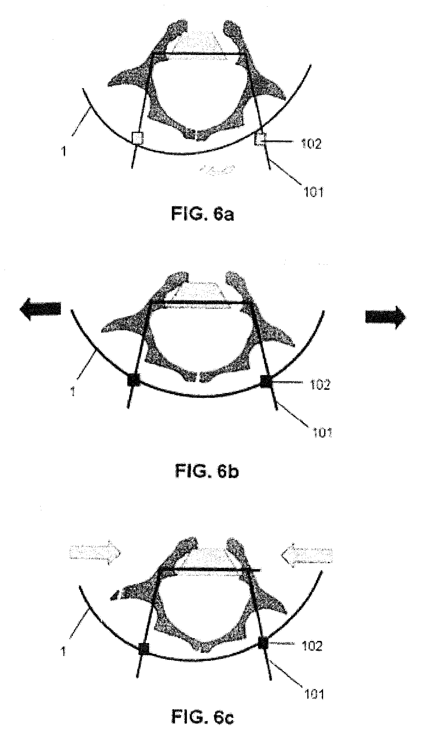

Figures 6a-6c show the way of using an external fixator (1) formed by a single piece such as that shown in Figure 1 using the turnbuckle (10). Firstly, Schanz pins (101) are inserted in the suitable place in the pelvic bone of the patient and the external fixator (1) is placed surrounding the patient's body, as shown in Figure 6a.

Next, the turnbuckle (10) of the invention is used for elastically "opening" the external fixator (1), tensing it, and Schanz pins (101) are then fixed to said fixator (1) by means of suitable ball and socket joints (102). The ball and socket joints (102) are depicted in this drawing with a darker tone to indicate that they are already closed, fixing the Schanz pins (101) to the external fixator (1). This situation in which the fixator (1) is still kept open by means of the turnbuckle (10) and the Schanz pins (101) are already coupled to said turnbuckle is shown in Figure 6b (the turnbuckle (10) is not depicted in the drawing).

Thirdly, the fixator (1) is uncoupled from the turnbuckle (10). The build-up elastic tension applies a force tending to cause the fixator (1) to recover its initial shape, a force that is transferred to the Schanz pins (101), and these pins in turn transfer said force to the bone fragments to which they are fixed. This results in the reposition of these bone fragments and, if the fixator (1) has been sufficiently tensed, a remaining force tending to "push" these bone fragments against one another. If this force is insufficient, it is possible to tense the fixator (1) again by reusing the turnbuckle (10), there being different methods for that purpose which are not part of the present invention.

Furthermore, the side arms (14a, 14b) of the turnbuckle (10) can comprise means which allow moving them along the central strut (13) in a controlled manner, for example, in a manner similar to the rotary control used for the rod to which the central support element (12c) is coupled. In that case, it would be possible to place the external fixator (1) with the inside of its central part supported on the central support element (12c) and the outer part of its ends supported on the side supports (12a, 12b), and then move the side arms (14a, 14b) inwards in a controlled manner, thereby causing the "closure" of the external fixator (1).

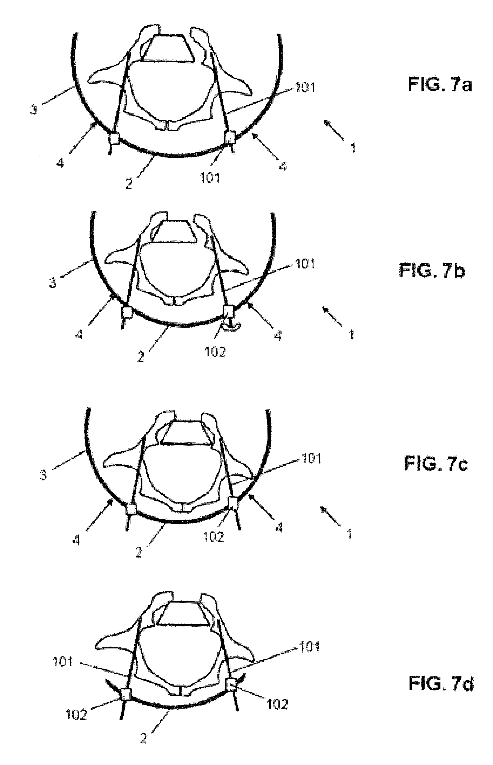

Figures 7a-7c describe the way of using the turnbuckle (10) for placing a detachable external fixator (1) such as that shown in Figure 2. Firstly, as depicted in Figure 7a, Schanz pins (101) are fixed to the pelvic bone fragments in the positions necessary in each case.

Next, the external fixator (1) of the invention is placed and tensed using the turnbuckle (10). Once the central portion (2) of the detachable fixator (1) has deformed outwards within its own plane, the Schanz pins (101) are fixed to said central portion (2) of the fixator (1) by means of suitable ball and socket joints (102). This situation is shown in Figure 7b.

The turnbuckle (10) is then removed, releasing the tension that had been applied to the central portion (2) of the fixator (1) through the end portions (3). The central portion (2) of the fixator (1) then tends to return to its initial shape and thereby applies a force on the Schanz pins (101), and therefore on the pelvic bone fragments, tending to bring said fragments back to their initial position. Figure 7c shows how the two pelvic ring fragments are almost back to their correct position.

Finally, the joints (4) connecting the central portion (2) of the fixator (1) to the end portions (3) are disconnected and the end portions (3) are removed. This results in the situation shown in Figure 7d, where only the central portion (2) remains connected to the Schanz pins (100). The patient has much more freedom of movements and recovery is therefore more bearable using the detachable fixator (1) of the invention.

## Claims

1. An external fixator (1) for pelvic fractures **characterized in that** it is formed by an elastic bar with an essentially C shape.

2. The external fixator (1) according to claim 1, having a curved elastic central portion (2) and two end portions (3), the end portions (3) being connected to the central portion (2) by means of a detachable joint (4) allowing transmission of tensions between said end portions (3) and the central portion (2).

3. The external fixator (1) according to claim 2, where the end portions (3) are curved.

4. The external fixator (1) according to claim 2, where the end portions (3) are straight.

5. The external fixator (1) according to any of claims 2-4, where the central portion (2) is made of carbon fiber.

6. The external fixator (1) according to any of claims 2-5, where the end portions (3) are made of carbon fiber or stainless steel.

7. The external fixator (1) according to any of claims 2-6, where the detachable joint (4) comprises first essentially planar prolongations (4a) projecting from the central portion (2) which are complementary with second essentially planar complementary prolongations (4b) projecting from the end portions (3), where the essentially planar surface of the first prolongations (4a) and second prolongations (4b) are perpendicular to the curvature of the fixator (1).

8. The external fixator (1) according to claim 7, where the planar prolongations (4a, 4b) comprise radial holes (4c) for inserting fixing means.

9. The external fixator (1) according to any of claims 2-6, where the detachable joint (4) is a male-female joint where a longitudinal projection of the central portion or side portions fits into a corresponding longitudinal hole of the side portions or central portion, respectively.

10. The external fixator (1) according to claim 9, where said longitudinal projection and longitudinal hole can be coupled to one another by means of threading.

11. An opening turnbuckle (10) for an external pelvic fixator (1) such as that described in any of the preceding claims, **characterized in that** it comprises two side support elements (12a, 12b) for the ends of the external fixator (1) and a central support element (12c) for the central part of the external fixator (1), where the central support element (12c) is longitudinally movable along a line perpendicular to the line joining said side support elements (12a, 12b).

12. The opening turnbuckle (10) according to claim 11, comprising:
a brace strut (13);
two side arms (14a, 14b) provided with the side support elements (12a, 12b), which can be slidably coupled to the brace strut (13); and
a central member (15) provided with the central support element (12c) which is coupled to the brace strut (13) between the two side arms (14a, 14b).

13. The opening turnbuckle (10) according to claim 12, where the two side arms (14a, 14b) are two bars having a first end slidably coupled to said brace strut (13) and a second end provided with the side support element (12a, 12b).

14. The opening turnbuckle (10) according to claim 13, where the side arms (14a, 14b) are straight bars.

15. The opening turnbuckle (10) according to claim 14, where the side arms (14a, 14b) are perpendicular to the brace strut (13).

16. The opening turnbuckle (10) according to any of claims 12-15, further comprising means for regulating in a controlled manner the position of the side arms (14a, 14b) along the central strut (13).

17. The opening turnbuckle (10) according to any of claims 12-16, where the central member (15) comprises a straight hollow bar inside which a rod provided at the end thereof with the central support element (12c) can slide longitudinally operated by means of a rotary control.

18. The opening turnbuckle (10) according to claim 17, where the central member (15) is perpendicular to the brace strut (13).

19. The opening turnbuckle (10) according to any of claims 12-18, where the brace strut (13) is a straight bar.

20. The opening turnbuckle (10) according to any of the preceding claims, where the central support element (12c) and side support elements (12a, 12b) comprise pulleys which allow sliding of the external fixator (1).
